# EUROPEAN PATENT SPECIFICATION

(11) **EP 0 457 901 B1**
(45) Date of publication and mention of the grant of the patent: **08.11.1995**
(21) Application number: 91902454.7
(22) Date of filing: 07.12.1990
(51) Int. Cl.: G01N 33/53, G01N 33/92, C12N 15/62

(54) **FREE FATTY ACID DETERMINATION**
BESTIMMUNG FREIER FETTSäUREN
DETERMINATION D'ACIDE GRAS LIBRE

(30) Priority: 12.12.1989 US 449972
(43) Date of publication of application: 27.11.1991
(73) Proprietor: LIDAK PHARMACEUTICALS, La Jolla, CA 92037 (US)
(72) Inventor: KLEINFELD, Alan, M., La Jolla, CA 92037 (US)
(74) Representative: Lehmann, Klaus, Dipl.-Ing.
(86) International application number: US9007202
(87) International publication number: WO9109310

(56) References cited:
- US-A- 4 491 631
- THE JOURNAL OF BIOLOGICAL CHEMISTRY vol. 263, no. 12, 25 April 1988, USA pages 5815 - 5819; J.C.SACCHETTINI ET AL.: 'The Structure of Crystalline Escherichia coli-derived Rat Intestinal Fatty Acid-binding Protein at 2.5-A Resolution'
- THE JOURNAL OF BIOLOGICAL CHEMISTRY vol. 262, no. 12, 25 April 1987, USA pages 5931 - 5937; J.B.LOWE ET AL.: 'Expression of Rat Intestinal Fatty Acid-binding Protein in Escherichia coli'
- MOLECULAR AND CELLULAR BIOCHEMISTRY, vol. 98, no. 1&2, 1990, pages 135 - 140; C.EVANS ET AL.: 'The chemical modification of cysteine-69 of rat liver fatty acid-binding protein (FABP): a fluorescence approach to FABP structure and function.'
- Biochemistry 32 (1993) 6275
- Biochim Biophys Acta 1081 (1991) 1
- Adv Protein Chem 45 (1994) 90
- J Biol Chem 269 (1994) 1

## Description

### Background of the Invention

This invention relates to research and clinical biochemical assays for a wide class of small hydrophobic molecules, wherein the analyte is caused to react with a specifically mutated and fluorescently modified, small-molecular-weight-binding protein, and thereby causes a detectable fluorescence signal. One specific application of the present invention is to assay for free fatty acids, either in whole blood, serum, food preparations, or various laboratory conditions using a suitably altered fatty-acid-binding protein (FABP).

Long chain free fatty acids (FFA) with acyl chains > 16 carbons are quantitatively the most important physiological energy source. While ubiquitous and essential for normal physiological function, FFA are also potent modulators of cellular activity, Karnovsky, M.J., Kleinfeld, A.M., Hoover, R.L. and Klausner, R.D. J. *Cell Biol*. *49*:1-6, 1982; Richieri, G. and Kleinfeld, A.M., *J. Immunol. 145:1024-1077*, 1990. There are, in fact, numerous indications that FFA levels uniquely reflect various states of health and disease. Variations in FFA levels have been reported in a number of pathologies including AIDS, ischemia, inflammation, diabetes, immune dysfunction, and cancer. Brown. R.E., Steele, R.W., Marmer, D.J., Hudson, J.L. and Brewster, M.A. *J. Immumol. 131*:1011, 1983; Hochachka. P.W. Science *231*:234, 1986: Levy, J.A. in *Basic and Clinical Immunology,* D.P. Stites. J.D. Stobo, H.H. Fudenberg, and J.V. Wells, eds.. Lange Medical Publications, Los Altos, CA, pp. 293-301, 1984; Reaven, G.M., Hollenbeck, C. Jeng, C.Y, Wu, M.S. and Chen, Y-D.I. *Diabetes 37*:1020, 1988; Tsuchiya, H., Hayashi, T., Sato, M., Tatsumi, M. and Takagi, N. *J. Chromatogr. 309*:43, 1984.

In specific instances, the concentration of FFA may be of significant importance in the diagnosis or treatment of disease or in studying the underlying biochemical or immunochemical causes or effects of disease. For example: FFA are believed to be important factors in the cause of ventricular arrhythmias during acute myocardial infarction, Makiguchi M, *Hokkaido Igaku Zasshi* (*JAPAN*) Jul 1988. 63 (4) p624-34. Significant differences in free fatty acids from normal levels in AIDS patients may be implicated in the pathophysiology of AIDS and could represent a good index of diagnosis and prognosis, Christeff, N.; Michon, C.: Goertz, G.; Hassid, J.; Matheron, S.; Girard, P.M.; Coulaud, J.P.; Nunez, E.A. ital *EUR. J. CANCER CLIN. ONCOL*.; 24(7), pp. 1179-1183 1988. Essential fatty acid (EFA) deficiency has been shown to protect against the glomerulonephritis in a murine model of systemic lupus erythematosus, Letkowith. J.B.; Jakschik, B.A.; Stahl, P.: Needleman, P., *J.* BIOL. *CHEM.*; 262(14), pp. 6668-6675 1987. Ambient plasma free fatty acid concentrations in non-insulin-dependent diabetes mellitus may be indicative of insulin resistance, Fraze, E.; Donner, C.C.; Swislocki, A.L.M.; Chiou, Y.-A.M.; Chen, Y.-D.I.; Reaven, G.M., *J. CLIN. ENDOCRINOL. METAB.*; 61(5), pp. 807-811 1985. Fatty acids have been implicated in the pathogenesis of thromboatherosclerosis, Tavella, M.; Mercuri, O.; de Tomas, M.E., *NUTR. RES.*; 5(4), pp. 355-365 1985. Depression of serum calcium may result from increased plasma free fatty acids, Warshaw, A.L.; Lee, K.-H.; Napier, T.W.; Fournier, P.O.; Duchainey, D.: Axelrod, L., *CASTROENTEROLOGY*; 89(4), pp. 814-820 1985.

Elevated levels of FFA have been found in human cancer patients and murine models (Lipaspi, A., Jeevanandam, M., Starnes, H.F., & Brennan, M.F., *Metabolism 36*:958. 1987; Iguchi, T., Takasugi, N., Nishimura, N., & Kusunoki, S. *Cancer Res*. *49:*821, 1989; Brown, R.E., Steele, R.W., Marmer, D.J., Hudson, J.L., & Brewster, M.A. *J*. *Immunol*. *131*:1001, 1983) and these elevated levels were shown to result in immunological deficiencies (Brown, R.E., Steele, R.W., Marmer, DJ., Hudson, J.L., & Brewster, M.A. *J*. *Immunol*. *131*:1001,1983).

In addition to their importance in disease, the measurement of FFA levels has important applications in a wide variety of biochemical, biophysical, cell biologic, and physiological research. These include studies of FFA transport (Storch, J. and Kleinfeld, A.M., *Biochemistry* 25:1717. 1986; Potter, B.J., Sorrentino, D. and Berk, P.D.,*Ann. Rev. Nutr*. 9:253, 1989), inter and intra-cellular signalling (Kim, D., Lewis, D.L., Graziadel, L., Heer. E.J., Bar-Sagi, D. and Clapham, D.E., *Natur*e *337*:557, 1989). and membrane structural perturbation (Karnovsky, M.J.. Kleinfeld, A.M., Hoover, R.L., and Klausner, R.D. *J*. *Cell Biol*. *49*:1, 1982.

Determination of FFA levels is also important in the food industry, since FFA often represent products of various stages of food breakdown, as in auto or enzymatically catalyzed triglyceride hydrolysis. Uji Y: Okabe H; Sakai Y; Noma A; Maeda M; Tsuji A, *Clin Chem* (*UNITED STATES*) Feb 1989, 35 (2) p325-6 (chemiluminescent determination of non-esterified fatty acids in serum).

While vital information about both normal and pathological physiology would accrue from the measurement of plasma levels of FFA, there are two essential barriers to obtaining this information. First, no method exists for measuring the aqueous phase concentration of unbound FFA. The un-esterified and unbound (as opposed to bound to serum albumin) FFA in the aqueous phase is, however. the active form of the FFA in both normal and pathologic states. Second, although the concentration of unbound FFA can be estimated, this can be done only with considerable effort. The unbound FFA concentration is estimated from the ratio of total serum FFA to total serum albumin. Once the total FFA and total albumin have been measured, the unbound FFA concentration is calculated using the FFA-albumin association coefficients (∼8 different sites/albumin molecule) determined from measurements of FFA partition between an albumin-water phase and heptane, Ashbrook, J.D., Spector, A.A., Santos, E.C. and Fletcher, J.E. *J. Biol. Chem. 250*:2233, 1975. Typical values calculated in this fashion yield concentrations in the range of 0.05 to 0.5 µM. Spector, A.A. and Fletcher, J.E. in *Disturbances in Lipids and Lipoprotein Metabolism*, J.M. Dietschy, A.M. Gotto and A. Ontko, eds., American Physiological Society, Bethesda, Maryiand, pp. 229-249, 1978. Even this estimate, however, can only be obtained with considerable effort since the determination of both total FFA and total albumin concentrations requires several very time-consuming and expensive steps. Determination of total FFA and albumin first requires the separation of plasma and cellular components in whole blood. This is done by centrifugation and decantation of the upper (plasma) phase. A radioactive or fluorescent ELISA assay can be used for determining the plasma concentration of albumin. Several approaches have been used to determine total FFA. In the most common, the lipid fraction must first be extracted from total plasma using, essentially, the method of Folch *et al*., Folch, J., Lees, M. and Stanley, G.H.S. *J. Biol. Chem. 226*:497, 1957. This method involves suspension of a quantity of plasma fluid in a solution of chloroform:methanol, centrifugation of this mixture and decantation of the supernatant, re-extraction of the residue with methanol:chloroform:water, centrifugation of this second mixture followed by decantation of the second supernatant and combination with the first. Chloroform is added to the combined supernatants, a two-phase mixture is produced by centrifugation, and the lower chloroform phase which contains the lipid is saved. At this stage most previous workers have concentrated the chloroform phase, derivatized the fatty acids to methyl esters and then performed chromatography to quantitate the various components. Baty, J.D. and Pazouki, S. *J*. *Chromat. 395*:403, 1987. It is possible, however, to determine the total FFA content of a heptane extract by adding a chloroform solution containing a divalent cation such as ⁶³Ni to the heptane extract. A two phase system is produced in which the upper phase contains complexes (probably micelles) of ⁶³Ni complexed with the anionic FFA and the total FFA concentration can be determined from the ⁶³Ni activity, Ho, R.J. *Anal*. *Biochem. 36*:105. 1970. A variation on these approaches involves the direct derivatization of FFA while in plasma, using visible-UV FFA reactive reagents (Miwa, H., Yamamoto, M., Nishida, T., Nunoi, K. and Kikuchi, M. *J*. *Chromat. 416*:237, 1987. The derivatized FFA complex is then extracted from plasma using organic solvents and FFA are then assayed using HPLC.

Another method for estimating lipid component is described by Imamura Shigeyuki, et al., U.S. Patent 4,491,631, wherein an enzyme having enoyl-coa hydratase activity, 3-hydroxyacyl-coa dehydrogenase activity and 3-ketoacyl-coa thiolase activity, all in the same enzyme, is produced by culturing the microorganism strain pseudomonas fragi b-0771 ferm-p no. 5701. and isolating the enzyme thus produced from the culture medium. The enzyme is useful in an assay method for a fatty acid component in a sample, which fatty acid is originally present in the sample or is liberated from a fatty acid ester in the sample. The assay is carried out by converting the fatty acid to acyl-coa, converting the thus-produced acyl-coa to dehydroacyl-coa; converting the thus-produced dehydroacyl-coa to hydroxyacyl-coa, converting the thus-produced hydroxyacyl-coa to ketoacyl-coa, converting the thus-produced ketoacyl-coa to acyl-coa and measuring the detectable changes in the reaction mixture.

These assay methods generally are complicated and time consuming, and are not readily carried out in any but a well-equipped reseach laboratory. Thus, there remains a need for an improved assay for free fatty acids in plasma, serum and blood.

### Summary of the Invention

This invention is embodied, in one application, to a method for determining unbound free fatty acids comprising the steps of mixing a solution suspected of containing free fatty acid with a reagent comprising a fluorescently modified fatty acid binding protein, detecting a change in fluorescence of the fluorescently modified fatty acid binding protein, and relating said change in fluorescence to the amount of free fatty acid in the solution. The change in fluorescence may be qualitative, e.g. a shift in wavelength, or quantitative, e.g. an increase or decrease in intensity upon binding.

The same method, as described hereinafter or with minor modifications such as are described and referred to, is suitable for use in detecting and measuring other hydrophobic molecules such as, for example, free cholesterol, steroids, hormonal steroids in particular such as estrogen, progesterone and testosterone, and retinoic acid.

In a preferred form, the method for determining unbound free fatty acids comprises mixing a solution suspected of containing unbound free fatty acid with a reagent comprising a fluorescently modified. cloned and mutagenized fatty acid binding protein which comprises binding sites which bind specifically to unbound free fatty acid, detecting a change in fluorescence of the fluorescently modified fatty acid binding protein and relating said change in fluorescence to the amount of unbound free fatty acid in the solution.

According to this invention, a technology is provided for determining plasma concentrations of unbound free fatty acids (ubFFA) in a single rapid step by detecting and quantifying the binding of ubFFA monomers to a fluorescently modified Fatty Acid Binding Protein (FABP). The protein to be used for this purpose is the 15.1 kDa intestinal FABP (I-FABP) which was originally obtained from the cytosol of rat small intestinal epithelium. I-FABP cloned and expressed from E. *coli* is fluorescently modified at particular sites such that, upon binding ubFFA, a readily detectable fluorescence change will occur.

The native protein was obtained with a vector containing the unmodified gene from which it was cloned and was expressed from E. coli. Oligonucleotide directed. site-specific mutagenesis is then used to modify the gene construct so that the modified construct can be fluorescently labeled at a particular site or sites. This modified construct is used to transfect E. coli and the mutagenized protein is expressed and purified.

In one particular embodiment, the native gene construct is modified to contain a codon for one or two cysteine residues. As an example, the cysteine residues are derivatized with dansyl aziridine, a sulfhydryl reagent, and the fluorescence changes of the dansylated I-FABP induced upon binding FFA are characterized. The invention is not limited to a dansyl derivative, since any fluorescent derivative which changes fluorescence in this configuration would be satisfactory.

With an appropriate fluorescent moiety, the method can be used with whole blood, thereby eliminating the need to separate blood into plasma and cellular components, to assay for serum albumin, to extract lipid from plasma, to carry out enzymatic reactions, to perform derivatization chromatography, or to carry out a phase separation-cationmicelle assay.

### Brief Description of the Drawings

Figure 1 depicts the fluoresence emission spectra of I-FABP (82) - ACRYLODAN titrated with oleic acid.

Figure 2 depicts the ratio of 497nm to 430nm emission intensities as a function of unbound oleic acid.

### Description of the Preferred Embodiments

The assay of this invention involves the single determination of the fluorescence intensity of fluorescent FABP added to whole blood, serum or any aqueous solutions. Moreover, this method directly determines the concentration of unbound free fatty acids (ubFFA), the physiologically relevant form of the molecule. The total FFA concentration could be calculated from the measured ubFFA and albumin concentrations.

The assay for ubFFA utilizes a suitably modified FABP. These are small (14-15 kDa) proteins widely distributed in many cells. especially intestinal epithelium, liver, adipose and cardiac tissue. FABP specifically bind long chain FFA, and they do this with an association constant (Kₐ) of about 10⁶ M⁻¹. Both the liver and intestinal form of the protein have a single FFA binding site. The specificity of the binding is fairly pronounced; Kₐ for FFA with chain lengths <14 carbons is negligible, and somewhat different proteins are required for binding sterols, retinol and other small hydrophobic molecules. The interference of other hydrophobic molecules is not usually a significant factor because of the above-described discrimination of the FABP and because the relative aqueous (of blood plasma) phase concentration of potential competitor molecules is negligible in comparison with long chain FFA.

The ability of FABP to discriminate in favor of long chain FFA has its origins in the unusual properties of the binding site. The x-ray structure of the I-FABP has recently been solved to about 2.5Å, and the solution was obtained with palmitic acid complexed with the protein, Sacchettini, J.C., Gordon, J.I. and Banaszek, L.J. *J. Biol*. *Chem. 263*:5815, 1988. This structure shows that the FFA is bound in a "clam shell"-like pocket formed by twelve strands of beta sheets. The FFA is bound so that its acyl chain is bent at the middle position (between carbon 6 and 8), suggesting a fairly constrained conformation, and among the amino acids which comprise the region around the bend there is a tryptophan residue (Trp₈₃). A fluorescence spectroscopic study of the liver form of this protein (expected to have a structure similar to I-FABP) and its interaction with FFA, Storch, J., Bass, N.M. and Kleinfeld, A.M. *J. Biol. Chem.264:*8708, 1989, using a series of long chain FFA in which an anthroyloxy moiety is ester linked to one of eight different positions along the acyl chain (n-AOFFA). directly demonstrated that the FFA is highly constrained at the 6-9 positions and that the intrinsic tyrosine fluorescence of the protein increases significantly upon binding natural (unmodified) long chain FFA.

The fluorescence responses observed in the above mentioned studies form the basis for the present invention. Upon binding to FABP in this highly constrained state. the protein itself undergoes a significant conformational change. This change is expected to be reflected in the alteration of tryptophan fluorescence. The alteration in Trp fluorescence itself cannot be used to detect FFA binding in plasma or whole blood, since the Trp fluorescence from serum proteins and cells would dominate the total emission. However, according to this invention, the addition of a suitable fluorescent moiety will undergo a fluorescence change which is distinguishable from background fluorescence or will allow the change in Trp fluorescence to be made distinguishable. The inventive assay of ubFFA involves adding modified FABP to an aqueous sample containing FFA and determining the change in fluorescence upon FFA interaction. FABP is an appropriate detector of plasma ubFFA levels since its Kₐ is on the same order as the expected ubFFA concentration in plasma (0.05 to >1 µM).

The background technology of the invention involves the following steps. Cloning of I-FABP from rat intestine from E. coli has been carried out previously and has been sequenced, cloned, and expressed in *E.coli* by previous workers, Alpers. D.H., Strauss. A.W., Ockner, R.K., Bass, N.M. and Gordon. J.I. *Proc. Natl*. *Acad*. *Sci*. *USA 81*:313, 1984; Lowe, J.B., Sacchettini, J.C., Laposata, M., McQuillan, JJ. and Gordon, J.I. *J. Biol. Chem. 262*:5931, 1987. Expression and protein purification have also been carried out, Sacchettini, J.C., Gordon, J.I. and Banaszek, L.J. *J. Biol*. *Chem.* 263:5815, 1988. The task of cloning is made especially easy by the great abundance of the protein, almost 8% of the cytosolic protein is FABP in some tissues.

A fluorescently derivatized mutant of I-FABP is next obtained. Since tyrosine fluorescence increases upon FFA binding, Storch, J., Bass, N.M. and Kleinfeld, A.M. J. *Biol*. *Chem*. *264:*8708, 1989, and the x-ray structure indicates that Trp₈₃ specifically interacts at the FFA bend, Sacchettini, J.C., Gordon, J.I. and Banaszek, L.J. *J. Biol*. *Chem. 263*:5815, 1988, then Trp₈₃ almost certainly undergoes a change in environment which in turn will alter the environment of the flanking residues at positions 82 and 84. Thus, either the direct emission of a dansyl group at positions 82 or 84 and/or the rate of resonance energy transfer between Trp₈₃ and dansyl₈₂ or dansyl₈₄, will change. Moreover. since residues 82 and 84 are directed away from the FFA binding site (on this β strand), modification of these sites will not affect FFA binding. It is known that fluorescence changes of dansyl aziridine conjugates of calcium binding proteins exhibit large changes in fluorescence upon calcium binding, Alexis, M.N. and Gratzer, W.B. *Biochem. 17*:2319, 1978. Modification of other residues of I-FABP and/or the other fluorescent groups may provide a more optimal response, and is within the scope of the invention.

Calibration procedures necessary to relate the observed fluorescence intensities to ubFFA concentrations and establish optimal conditions for both plasma and whole blood analysis are established by recognized procedures, comparing signal strength to known FFA content. FFA blood levels in normal individuals and patients with various pathologic disorders are then determined as described.

### Technical Details

***Cloning of I-FABP from rat intestine.*** Cloning was carried out using a cDNA library from neo-natal rat intestine purchased from STRATEGENE (11099 North Torrey Pines Rd., La Jolla, CA 92037). The library is carried by the LAMBDA ZAP II vector and was used with the E. coli host strain XL1-Blue which is supplied with the library. The recombinant clone was identified with an oligonucleotide probe based upon the published sequence of the I-FABP DNA (Lowe, J.B., Sacchettini, J.C., Laposata, M., McQuillan, J.J. and Gordon, J.I. *J*. *Biol Chem. 262*:5931, 1987) and sequencing of 5′ and 3′ regions was used to verify the identity and completeness of the gene. The amino acid sequence of this clone was found to be identical to that described earlier by Lowe et al (1987. J. Biol. Chem. 262:5931). The I-FABP gene was removed from the lambda Zap vector and inserted into a PET expression vector (PET11D) purchased from Novagen and the protein was expressed using the E. coli strains (BL21) also from Novagen (Madison, WI, USA).

***Isolation and Purification of I-FABP***. I-FABP is isolated from *E*. *coli* and purified as described, Lowe, J.B., Sacchettini, J.C., Laposata, M., McQuillan, J.J. and Gordon, J.I. *J. Biol*. *Chem. 262:*5931, 1987. In this procedure cells, after a 3-hour culture, are lysed. centrifuged to remove cell debris and the pellet frozen at -70°C. The frozen pellet was allowed to thaw in the presence of a lysing buffer composed of 50mM Tris, 0.5mM EDTA, 0.5mM PMSF, 0.1% Triton® X100, at pH 8.0. This was centrifuged at 20,000 g. for 45 minutes and the pellet was discarded. The lysate was then subjected to two rounds (50% and 70%) of ammonium sulfate precipitation and the supernatant from the 70% material was desalted and concentrated using an Amicon ultrafiltration apparatus with a YM10 filter. The filtrate was then subjected to DEAE-Sephadex® (A50), followed by Sephadex® G-75 chromatography and the low molecular weight fractions were saved. This material gives a single band on 12% PAGE and an amino acid analysis of this material is consistent with the amino acid sequence of I-FABP. The addition of FFA (oleate, stearate, or palmitate) reduced the tryptophan fluorescence of the native protein by as much as 40%. This procedure yields about 70 mg/1.0 liter fermentation of a single band running at about 14.6 kDa on SDS-PAGE.

***Modification of I-FABP by site-directed mutagenesis***. Site-directed mutagenesis is used to modify the protein at discrete residues by introducing one or two cysteine residues at positions which the 3° structure and our own studies indicate will probably not interfere with FFA binding but will likely exhibit an environmental change as a consequence of FFA binding. This change, in turn, will manifest itself as a measurable alteration in fluorescence intensity of a fluorescent moiety derivatized to the cysteine's SH group. Site directed mutagenesis was used to introduce a cysteine residue at positions 82 or 84, in place of the threonine residues that are normally at this position. Mutagenesis is carried out by annealing a synthetic primer with a single mismatched base to the above derived expression vector. The primer is extended and ligated *in vitro,* and the resulting heteroduplex is used to transfect the host strain. The transfected host is screened for the mutated DNA by hybridization with the mutating oligonucleotide. Mutated plasmid DNA is isolated, re-transformed into the host strain, and its structure confirmed by sequencing the recombinant DNA in regions defined by probes flanking the mutated region. This modified protein was expressed in the same system and purified by the same methods as for the native protein. Typical yields of purified protein are approximately 50 mg/liter cell culture.

***Fluorescence properties of a mutated and fluorescently derivatized I- FABP***. The mutated I-FABP protein is fluorescently derivatized by inserting a fluorescent group (which reacts specifically with free SH groups) at the newly created cysteine residue. The cysteine residue is inserted at positions 82 (replacing a threonine residue) and/or at residue 84 (also a threonine residue) since both these positions face outward from the FFA binding pocket but are in close proximity to the tryptophan at position 83 (about 3.5Å lateral separation along the β strand and about 10Å vertical separation between the centers of the tryptophan and fluorescent residues). It is postulated, since the tryptophan fluorescence increases upon FFA binding and the x-ray structure indicates that Trp₈₃ specifically interacts at the FFA bend, that Trp₈₃ may rotate into a lower dielectric environment which in turn will induce a change in the environment of residues 82 and 84. Thus. either the direct emission of the fluorescent group and/or the rate of resonance energy transfer between Trp₈₃ and the fluorescent group is postulated to change.

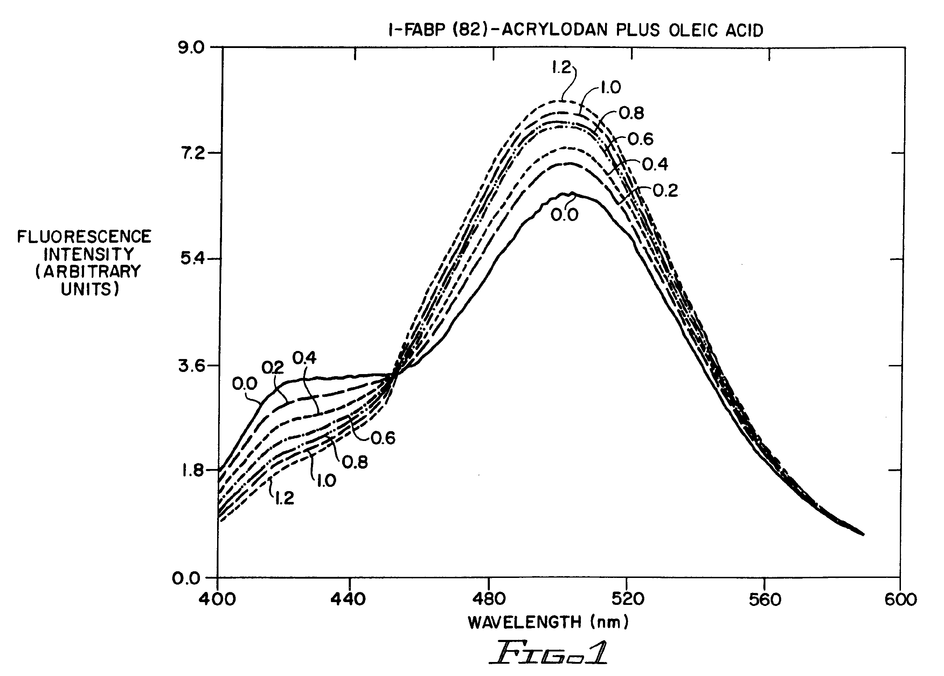

In a specific example, the dansyl derivative ACRYLODAN® (6-acryloly-2-dimethylamenonaphthalene) was used to modify the mutated FABP. A most valuable and unexpected phenomenon was discovered in carrying out the invention as described. Reference is made to Figure 1 which depicts the fluoresence emission spectra of I-FABP (82) - ACRYLODAN titrated with oleic acid. The emission of I-FABP, in which T-82 has been mutated to Cys-82 and this cysteine was derivatized with ACRYLODAN, is shown as a function of increasing concentrations of unbound oleic acid. The protein was dissolved at a concentration of 0.5µM in an aqueous buffer consisting of 50mM Tris, 150mM KCl, 0.5mM DDT, 0.02% Na Azide, Ph 7.4. Oleic acid was added as a complex with small unilamellar vesicles of dimyristoylphosphatidylcholine (DMPC) The vesicle concentration was fixed at [DMPC] = 500µM, total oleic acid varied between 0 and 100 µM, and unbound oleic acid was determined using a partition coefficient of 70,000. The unbound oleic acid for each spectrum are indicated in Figure 1. These spectra were obtained using an SLM4800 fluorometer in the ratio mode, with excitation wavelength = 400nm, excitation slits at 4nm and emission at 16nm.

Thus, addition of long chain FFA to this modified protein caused a monotonic spectral change that was isosbestic with increasing FFA concentrations. These changes occur in the same concentration range as those observed in the native protein and suggest that the mutagenesis and fluorescent modification did not significantly alter the FFA binding characteristics of the protein. More importantly, these results indicate that the ratio of fluorescence emissions at 430 and 497nm may provide a direct measure of the concentration of unbound FFA.

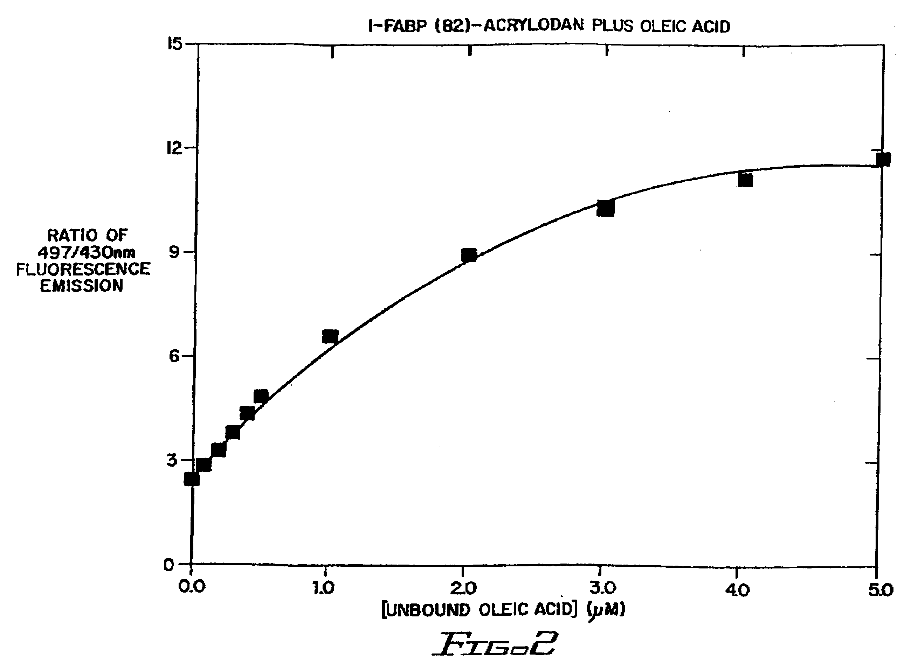

As shown in Figure 2, the ratio of 497nm to 430nm emission intensities varies monotonically with increasing unbound FFA concentration. The zero FFA value is about 2.5 and the maximum value, at saturation (∼ 4µM oleic acid) is about 18.

The mutant protein derivatized with dansyl aziridine also shows an altered fluorescence response with added FFA and is considered advantageous when a shorter wavelength emitting probe is desired. A variety of fluorescent labels are available for use at various wavelengths.

There are numerous indications that FFA levels uniquely reflect various states of health and disease. Measurements of FFA levels are not performed on a regular basis simply because there are no suitable assays. In addition, since elevated levels are associated with various pathologies including ischemia, inflammation, diabetes, AIDS and cancer, monitoring blood levels could be used to directly and rapidly monitor the progress of treatment. The present invention is readily adaptable to measurements of the plasma levels of other hydrophobic molecules including, for example, free cholesterol, hormonal steroids such as estrogen, progesterone and testosterone, and retinoic acid. The method of this invention provides a much faster and less expensive alternative to current methods. The fluorescenated FABP will also have wide applicability in studies of intra- and inter-cellular fatty acid transport, in models of inflammatory response, and in enzymatic assays such as those for various lipases. The present invention permits the differential assay of different chain length FFA and different degrees of saturation in FFA, as both different chain lengths and degrees of saturation can be detected by the method described. Industrially, the invention will be useful in detecting lipid degradation in food preparations.

### Industrial Application

The primary commercial application of this technology would be its inclusion in routine blood assay for lipids. steroids and related compounds, in assays involved in commercial production of certain food preparations, and in assays used in research in biochemistry and cell biology.

## Claims

1. An assay for the determination of free fatty acid, characterized in that
the concentration of unbound free fatty acid in aqueous phase is determined by carring out a method comprising the steps of: mixing the aqueous phase suspected of containing unbound free fatty acid with a reagent that consists essentially of fatty acid binding protein that
(a) binds specifically to unbound aqueous phase free fatty acid,
(b) has been labeled with a fluorescent moiety, and
(c) exhibits a measurably different fluorescence when unbound in aqueous phase than when bound in aqueous phase to theretofore unbound free fatty acid;
measuring the fluorescence difference resulting from the binding of the fatty acid binding protein to the theretofore unbound free fatty acid; and
deriving the concentration of unbound free fatty acid in said aqueous phase from said measured fluorescence difference.

2. The method of Claim 1 characterized in that the fluorescence difference is measured at two wavelengths.

3. The method of Claim 2 characterized in that the fluorescence difference is measured by measuring the ratio of fluorescence at two wavelengths.

## Patentansprüche

1. Test zur Bestimmung freier Fettsäuren, dadurch gekennzeichnet, daß die Konzentration von ungebundenen freien Fettsäuren in wäßriger Phase durch Durchführung eines folgende Stufen umfassenden Verfahrens bestimmt wird: Vermischen der wäßrigen Phase, von der angenommen wird, daß sie ungebundene freie Fettsäuren enthält, mit einem Reagens, das im wesentlichen aus einem Fettsäuren bindenden Protein besteht, das
(a) spezifisch eine Bindung mit ungebundenen, in wäßriger Phase vorliegenden, freien Fettsäuren eingeht,
(b) mit einem fluoreszierenden Rest markiert worden ist und
(c) in ungebundenem Zustand in wäßriger Phase eine meßbar unterschiedliche Fluoreszenz im Vergleich zu dem Zustand aufweist, in dem es in wäßriger Phase an eine bisher ungebundene, freie Fettsäure gebunden ist;
Messen der Fluoreszenzdifferenz, die sich aus der Bindung des fettsäurebindenden Proteins an die bisher ungebundene, freie Fettsäure ergibt; und
Ableiten der Konzentration von ungebundenen freien Fettsäuren in der wäßrigen Phase aus der gemessenen Fluoreszenzdifferenz.

2. Verfahren nach Anspruch 1, dadurch gekennzeichnet, daß die Fluoreszenzdifferenz bei zwei Wellenlängen gemessen wird.

3. Verfahren nach Anspruch 2, dadurch gekennzeichnet, daß die Fluoreszenzdifferenz durch Messen des Verhältnisses der Fluoreszenz bei zwei Wellenlängen gemessen wird.

## Revendications

1. Analyse pour la détermination d'acide gras libre,
caractérisée en ce que la concentration d'acide gras libre non-lié dans la phase aqueuse est déterminée par l'exécution d'une méthode comportant les étapes : mélanger la phase aqueuse soupçonnée de contenir de l'acide gras libre non-lié avec un réactif qui consiste essentiellement en une protéine liant l'acide gras, qui
(a) se lie spécifiquement à l'acide gras libre non-lié en phase aqueuse,
(b) a été marquée par un moyen fluorescent, et
(c) présente une fluorescence différente mesurable lorsqu'elle est non-liée dans la phase aqueuse que lorsqu'elle est liée dans la phase aqueuse audit acide gras libre non-lié;
mesurer la différence de fluorescence résultant de la liaison de la protéine liant l'acide gras audit acide gras libre non-lié; et
dériver la concentration de l'acide gras libre non-lié dans ladite phase aqueuse de ladite différence de fluorescence mesurée.

2. Méthode selon la revendication 1, caractérisée en ce que la différence de fluorescence est mesurée à deux longueurs d'ondes.

3. Méthode selon la revendication 2, caractérisée en ce que la différence de fluorescence est mesurée en mesurant le ratio de fluorescence à deux longueurs d'ondes.
